# EUROPEAN PATENT SPECIFICATION

(11) **EP 3 871 686 B1**
(45) Date of publication and mention of the grant of the patent: **09.07.2025**
(21) Application number: 19820861.3
(22) Date of filing: 23.10.2019
(51) Int. Cl.: A61K 38/38, A61P 25/28, A61K 45/06

(54) **USE OF LOW VOLUME PLASMA EXCHANGE FOR TREATING THE MIDDLE STAGES OF ALZHEIMER'S DISEASE**
VERWENDUNG EINES PLASMAAUSTAUSCHS MIT NIEDRIGEM VOLUMEN ZUR BEHANDLUNG VON MORBUS ALZHEIMER IM MITTLEREN STADIUM
UTILISATION D'UN ÉCHANGE PLASMATIQUE À FAIBLE VOLUME POUR LE TRAITEMENT DE LA MALADIE D'ALZHEIMER DANS LES STADES MODÉRÉS

(30) Priority: 25.10.2018 EP 18202720
(43) Date of publication of application: 01.09.2021
(60) Divisional application: 25170160.3
(73) Proprietor: Grifols Worldwide Operations Limited, Dublin 22 (IE)
(72) Inventor: GRIFOLS ROURA, Victor, 08022 Barcelona (ES); PAEZ REGADERA, Antonio Manuel, 08174 Sant Cugat Del Valles (ES); NUÑEZ DOMENECH, Laura, 08174 Sant Cugat Del Valles (ES)
(74) Representative: Durán-Corretjer, S.L.P.
(86) International application number: PCT/IB2019/001145
(87) International publication number: WO 2020/084346

(56) References cited:
- BOADA M; RAMOS-FERNÁNDEZ E; GUIVERNAU B; MUÑOZ F J; COSTA M; ORTIZ A M; JORQUERA J I; NÚÑEZ L; TORRES M; PÁEZ A: "Treatment of Alzheimer disease using combination therapy with plasma exchange and haemapheresis with albumin and intravenous immunoglobulin: Rationale and treatment approach of the AMBAR (Alzheimer Management By Albumin Replacement) study.", NEUROLOGIA, vol. 71, no. 7, 9 July 2014 (2014-07-09) - 9 July 2014 (2014-07-09), pages 473 - 481, XP002790386
- BOADA ET AL: "Changes in cognitive status of Alzheimer's disease patients treated with plasma exchange and replacement with human albumin plus immunoglobulin. Interim results of the AMBAR trial.", ALZHEIMER DEMENTIA, vol. 12, 2016 - 2016, pages P419 - P420, XP002790387

## Description

The present invention relates to the medical field, in particular to the use of low volume plasma exchange for the treatment of Alzheimer's Disease in middle stages.

Alzheimer's disease (AD) is the most common cause of dementia in the adult. The presence of intracellular neurofibrillary tangles (NFT) of phosphorylated tau protein deposits as well as amyloid plaques formed from extracellular aggregates of amyloid β peptides (Aβ) are hallmarks of AD pathology. Although both NFT and amyloid deposits are suspected to be responsible for cell death in the AD brain, the initial biological trigger of the pathology has not been fully elucidated. The amyloid cascade hypothesis has been widely studied to explain the pathogenesis observed in AD. It has served as a target for many drug development programs in search of an agent with disease modifying properties. The hypothesis proposes that production and deposition of amyloid plaques in the brain drives AD pathogenesis.

Large amounts of Aβ is neurotoxic to cells in a variety of ways, including apoptosis activation and the induction of oxidative stress associated with the production of free radicals, which led to altered oxidative metabolism. The pathologic metabolism leading to Aβ peptide accumulation in the brain is initiated in the amyloid precursor protein (APP), which is processed by several different proteases (secretases) following either a constitutive non-amyloidogenic pathway or a pathogenic amyloidogenic pathway. In the amyloidogenic pathway, APP is cleaved by β-secretase to release a soluble N-terminal fragment (sAPP-β) and a membrane-bound C-terminal fragment (C99), which in turn is cleaved by γ-secretase to produce soluble amyloid precursor protein intracellular domain (AICD) and a heterogeneous generation of insoluble Aβ peptides, with Aβ₁₋₄₀ and Aβ₁₋₄₂ being most common. Hydrophobic properties of Aβ facilitate the formation of amyloid plaque in the brain, particularly of Aβ₁₋₄₂ because it is less soluble and more toxic relative to Aβ₁₋₄₀. Conversely, in the non-amyloidogenic pathway, APP is constitutively cleaved by α-secretase within the Aβ sequence; therefore, the formation of amyloidogenic Aβ is prevented.

There are only symptomatic treatments approved for the treatment of AD, including cholinesterase inhibitors and N-methyl-d-aspartate (NMDA) receptor antagonists. Therapies to prevent the accumulation of amyloid deposits or to reduce the existing plaque are currently investigated for the treatment of AD, and several molecular targets of the amyloidogenic pathway are being or have been tested. Hence, interfering with factors that regulate the top upstream APP production may affect intracellular levels of APP and thus reducing the overall levels of Aβ.

Similarly, inhibition or modulation of major players involved in the neurotoxic Aβ-generating such as β-secretase and γ-secretase appear to be key therapeutic targets against AD. Alternatively, downstream strategies targeting amyloid deposits in brain tissue may inhibit Aβ aggregation or disrupt the already formed plaque. Finally, there is the clearance of Aβ using both passive and active immunotherapies (direct use of anti-Aβ monoclonal antibody, and stimulation of the immune system through vaccination with Aβ peptide fragments, respectively).

Unfortunately, clinical trials with small molecule pharmacotherapy and immunotherapies to reduce brain Aβ have not shown efficacy. Persistent failure has led investigators to develop new therapeutic strategies for AD aimed at lowering Aβ accumulation in the brain by changing the transportation of Aβ through the blood-brain barrier.

Therefore, there is still a need of succesful treatments of AD. The present inventors has conducted a clinical trial involving 496 patients with mild to moderate AD from centers in 41 sites, 19 in Spain and 22 in the United States, in which patients received different doses of albumin by therapeutic plasma exchange and low volume plasma exchange (LVPE), optionally combined with intravenous immunogliobulin (IVIG), and surprisingly found that change from baseline in the cognitive scores as measured with the Alzheimer's Disease Assessment Scale in cognition (ADAS-Cog scale), (Rosen WG, Mohs RC, Davis KL. A new rating scale for Alzheimer's disease. Am J Psychiatry. 1984;141:1356-64) and the Alzheimer Disease Cooperation Studies-Activities of Daily Living (ADCS-ADL inventory) (Galasko D, Bennett D, Sano M, Ernesto C, Thomas R, Grundman M, et al. An Inventory to Assess Activities of Daily Living for Clinical Trials in Alzheimer's Disease. Alzheimer Dis Assoc Disord. 1997;11:33-9) in patients with mild and moderate AD is improved. Surprisingly, said improvement is enhanced in patients with moderate AD. The treatment regime of LVPE requires smaller volumes than traditional therapeutic plasma exchange, shorter duration time and a single venipuncture, decreasing patient discomfort, and reducing adverse effects of the treatment. Preliminary results of the trial have been published in Boada et al.; Alzheimer Dementia, vol.12,2016,pages 419-420.

To aid understanding, the present invention is described in greater detail below, with reference to the attached figures, which are presented by way of example, and with reference to illustrative but nonlimiting example.
Figure 1 is an schematic view of the clinical trial interventions according to the present invention (TPE: therapeutic plasma exchange; LVPE: low volume plasma exchange; F: Flebogamma 5 % DIF [Intravenous immunoglobulin]; A: Albumin 5 %-20 % [albumin]; S: sham treatment; B: baseline visit; IV: intermediate visit: FV: final visit).
Figure 2 is a graph illustrating the ADAS-Cog change from baseline during time in the four groups of patients included in the clinical trial.
Figure 3 is a graph illustrating the ADCS-ADL change from baseline during time in the four groups of patients included in the clinical trial.
Figure 4 is a graph illustrating the ADAS-Cog change from baseline during time in all treated patients compared with the control group.
Figure 5 is a graph illustrating the ADCS-ADL change from baseline during time in all treated patients compared with the control group.
Figure 6 is a graph illustrating the ADAS-Cog change from baseline during time in treated patients with mild AD (MMSE 22-26) compared with the control group.
Figure 7 is a graph illustrating the ADCS-ADL change from baseline during time in treated patients with mild AD (MMSE 22-26) compared with the control group.
Figure 8 is a graph illustrating the ADAS-Cog change from baseline during time in treated patients with moderate AD (MMSE 18-21) compared with the control group.
Figure 9 is a graph illustrating the ADCS-ADL change from baseline during time in treated patients with moderate AD (MMSE 18-21) compared with the control group.

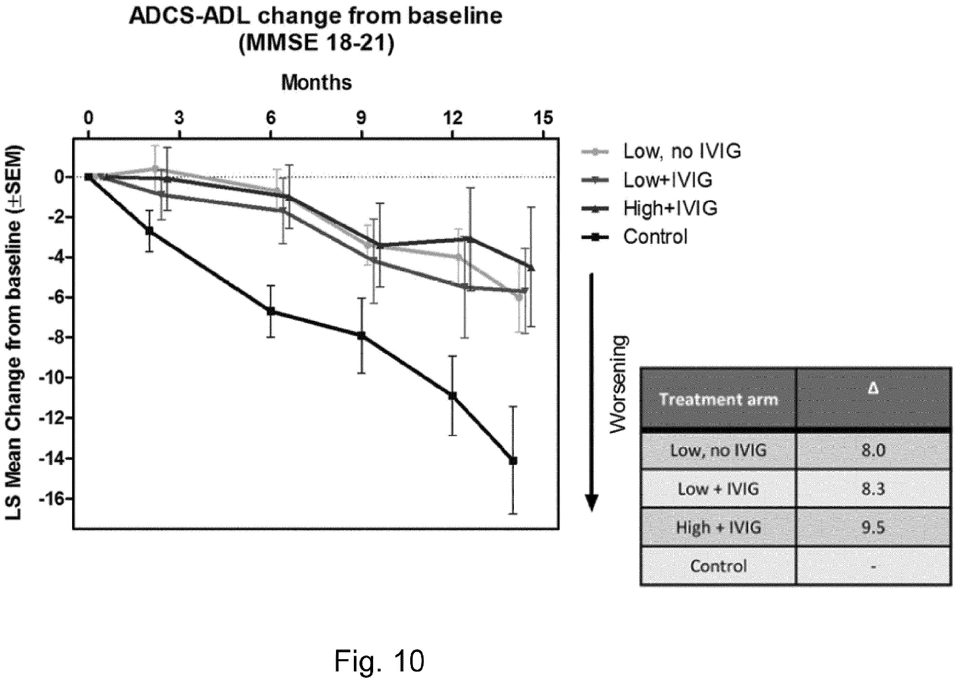

Figure 10 is a graph illustrating the ADCS-ADL change from baseline during time in patients with moderate AD (MMSE 18-21) of the three treated groups compared with the control group.
Figure 11 is a graph illustrating the levels of Aβ₁₋₄₂ in CSF at the end of each of the two treatment periods with all treated patients (A), in patients with mild AD (MMSE 22-26) (B) and with moderate AD (MMSE 18-21) (C).
Figure 12 is a graph illustrating the levels of T-tau (A) and P-tau (B) in CSF at the end of each of the two treatment periods in patients with moderate AD (MMSE 18-21).

The present invention refers to a composition comprising human albumin at a concentration between 5 % (w/v) and 25 % (w/v) for use in the treatment of moderate Alzheimer's Disease (AD) by low-volume plasma exchange (LVPE) as defined in claim 1.

As used herein, the term "low-volume plasma exchange" refers to plasma exchange treatments involving blood volumes between 600 mL - 900 mL.

As used herein, the term "mild AD" refers to individuals having a Mini-Mental State Examination (MMSE) score at baseline visit (Folstein MF, Folstein SE, McHugh PR. Mini-mental state, A practical method for grading the cognitive state of patients for the clinician. J Psychiatr Res. 1975;12:189-98) of 22 to 26.

As used herein, the term "moderate AD" refers to individuals having a MMSE score at baseline visit of 18 to 21.

In one embodiment, the treatment of the present invention comprise in addition the administration of intravenous immunoglobulins (IGIV). Preferably between 10 g and 20 g of immunoglobulins is used for replacement in some round of LVPE but never administered at the same time as albumin.

According to particular embodiments, each round of LVPE involves automated plasma exchange using a single venipuncture. Any extracorporeal plasma exchange device known in the art may be used in accordance with the present invention. For example, the device may include an entrance port for receiving the whole blood from a patient, means for separating plasma from the cellular components of the blood, and a means for returning the cellular components of the blood through the entrance port from where the blood cellular components exited the device, and an exit port from where the plasma exits the device. Preferably, the means for separating plasma from the cellular components of the blood are centrifugation means (i.e., a centrifuge). More preferably, the means for separating plasma from the cellular components of the blood are filtration means (i.e., a filter such as that used in double filtration plasma exchange).

The treatment regime comprises administering twelve rounds of LVPE to the patient.

Before the treatment regime of LVPE previous rounds of conventional therapeutic plasma exchange (TPE) are carried out. Said TPE regime is conducted at a frequency of 1 TPE per week, at least during 6 weeks. The term "TPE" refers to a plasma exchange in which 2500 to 3000 mL of patient's plasma was replaced with the same volume of a replacement fluid.

Each subsequent round of LVPE is conducted 30 days after the previous round.

According to particular embodiments, between 10 g and 40 g of albumin can be used for replacement in each round of LVPE, preferably between 20 g to 40 g of albumin in each round of LVPE.

### Example 1. Clinical trial

### Global study design

The clinical trial was a multicenter, randomized, blinded and placebo-controlled, parallel-group trial with a enrollment of 496 patients with mild to moderate AD from centers in 41 sites, 19 in Spain and 22 in the United States. The study included four groups of patients: three PE treatment groups receiving different doses of albumin and IVIG, and one control (placebo) group. Control patients were subjected to simulated PE through a noninvasive procedure (sham), that mimics plasma exchange but with neither fluid is replaced nor albumin or IVIG administered. The patients, caregivers and raters were blinded as to the therapy received. A single code was used for the anonymization of patients.

The study strictly follows the ethical standards adopted by the XVIII World Medical Assembly Declaration of Helsinki (and subsequent revisions) as well as the European Union standards of Good Clinical Practice (GCP) relating to trials involving drug products. Institutional Review Board (IRB) or Ethics Committee from the sites and the health authorities from both countries have approved the protocol, the informed consent form, and the patient information sheets.

The safety of the intervention was closely monitored. All serious and/or unexpected adverse reactions as well as any additional information that may alter the study design or entail patient risk were reported.

### Selection of study population

Participants in the clinical trial were men or women between 55-85 years of age at the time of signing of the informed consent document. They must have had a diagnosis of AD according to the National Institute of Neurological and Communication Disorders and Stroke and the Alzheimer's Disease and Related Disorders Association (NINCDS-ADRDA) criteria, have a Mini-Mental Status Examination (MMSE) score from 18 to 26, and a treatment with acetylcholinesterase-inhibitors (AchEls) and/or memantine with the previous three months at a stable dose. In addition, patients were free of cerebrovascular disease, evidenced by a brain computed axial tomography (CAT) or magnetic resonance imaging (MRI) study obtained in the 12 months prior to screening. An MRI required during the screening period is used to rule out any finding that could affect patient safety such as microhemorrhages, infarction, hematoma, stroke, or brain tumors including meningioma. Exclusion criteria include any condition in which plasma exchange is contraindicated or not feasible such as behavioral disorders, difficult venous access, or abnormal coagulation parameters or replacement products cannot be administered such as a history of frequent adverse reactions or thromboembolic complications associated with blood components, particularly hypersensitivity to albumin or allergies to any of the components of Albutein^{®} or Flebogamma^{®} DIF. Other exclusion criteria due to effect on patient's safety include IgA deficiency; low hemoglobin (< 10 g/dL); high plasma creatinine (> 2 mg/dL); uncontrolled hypertension (systolic ≥160 mm Hg, diastolic ≥100 mm Hg, despite regular treatment during the last 3 months), liver disease (GPT >2.5 x ULN, or bilirubin > 2 mg/dL), heart diseases, illness with less than one year of expected survival, drug or alcohol abuse; and pregnancy or nursing.

### PE interventions

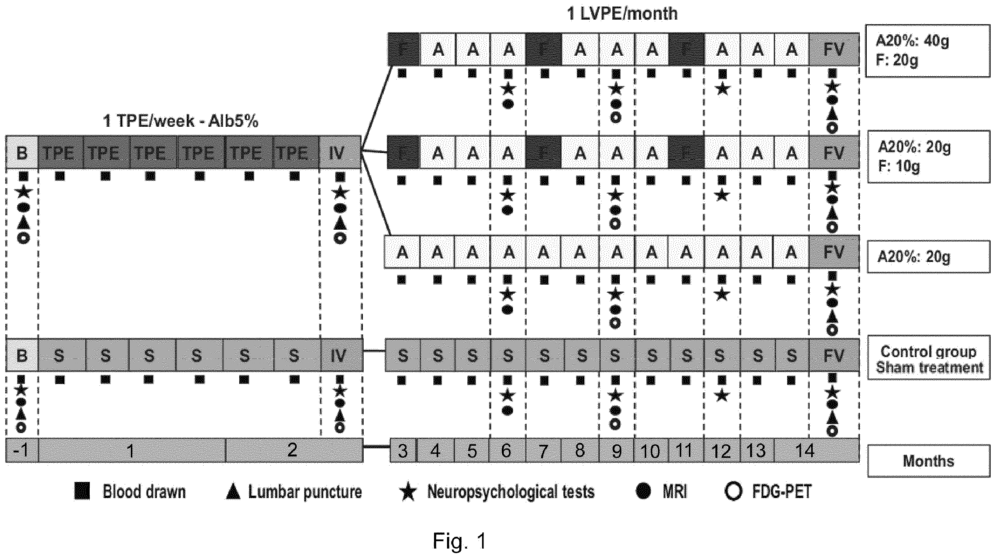

After the screening visit, the patients were randomized to one of the three treatment groups or the control (sham) group according to a [1:1:1:1] scheme. The intervention regime included a first 6-week stage of intensive treatment with one session of conventional therapeutic plasma exchange (TPE) per week, where all the patients assigned to any of the treatment groups follow the same treatment regime, followed by a second 12-month stage of maintenance treatment with one session of low-volume plasma exchange (LVPE) per month, as summarized in Figure 1. Between these two treatment periods, there was an intermediate visit to assess patient's status and collect additional study related data. At the corresponding PE sessions sufficient EDTA-blood was collected for all the laboratory tests. CSF samples were collected before and after of both treatment periods for all the laboratory tests. Aliquots of both plasma and CSF were stored at -70 ºC for future analysis. At the end of the maintenance period, the patient had the final visit which evaluates the same variables as the screening and intermediate visits.

During TPE, 2500 to 3000 ml of patient's plasma was replaced with the same volume of Albutein^{®} 5 % (GRIFOLS, S.A.) while during LVPE only 650 mL to 880 mL of patient's plasma was replaced with 100 mL or 200 mL of Albutein^{®} 20 % (GRIFOLS, S.A.), depending on the treatment arm randomization. There were three arms of LVPE: in one arm only 20 grams of Albutein^{®} 20 % were used for replacement in LVPE, while in the other two arms Albutein^{®} 20 % replacement (20 or 40 grams) was alternated with Flebogamma^{®} DIF 5 % (10 or 20 grams; GRIFOLS, S.A.).

TPE was performed using a commercial continuous flow cell separator with either centrifugation or filtration based technology. Either a peripheral (e.g., radial/cubital vein) or central access (e.g., subclavian/jugular vein) was used based on the individual characteristics of the patient. Venous access implantation and maintenance were carried out according to the standard procedures used in each center and the correct placement of a central catheter is always confirmed by a chest X-ray. The volume of removed and replaced plasma depended on patient's characteristics (i.e., sex, height, weight and hematocrit). It was approximately 35-45 mL/kg, corresponding to a volume of 2500-3000 mL. LVPE was carried out through a peripheral line by means of a prototype apheresis device based on the Auto-C^{™} device (Fenwal Inc, Lake Zurich, IL, USA) or the Aurora^{™} device (Fresenius Kabi, Bad Homburg, Germany) , the newer version of Auto-C^{™}. The removed plasma volume is like to that of a plasma donation (650-880 mL) and depended on patient's weight. The volume of infused albumin is consistent with treatment arm as described above. Normal saline was used when there is a negative balance between the volume of removed plasma and infused albumin.

For the control group (sham TPE), the tip of a cut catheter was stitched to a colostomy adhesive patch (acting as a "second skin") which was placed on the subclavicular or jugular region. Then, the patch with the catheter tip stitched was covered with gauze dressing and an adhesive film. The cut catheter was of characteristics similar to the catheters used in the treatment group. A conventional PE device was loaded with a saline solution colored with intravenous iron mimicking plasma and was working in a closedcircuit manner without any fluid interchange between the device and the subject. For the control group sham LVPE, an Autopheresis-C- or Aurora-based prototype provided an apparently and realistic working status, in which expired human blood from a local blood bank was circulated in a closed-circuit manner.

### Outcome Measures

The coprimary efficacy variables were: i) the change from baseline in the cognitive scores as measured with the ADAS-Cog scale; and ii) the change from baseline in the functional scores measured by the ADCS-ADL inventory. In both cases there were 6 measurements performed: one at week -3, -2 or -1 (baseline), one at week 7-8 (end of intensive treatment period), and one at months 6, 9, 12 and 14 (end of maintenance treatment period). Fourteen months was the end point for the primary efficacy analysis (see Figure 1). Secondary efficacy variables as change in plasma levels if Aβ₁₋₄₀ and Aβ₁₋₄₂ between before and after each PE, changes in cerebrospinal fluid (CSF) levels of Aβ₁₋₄₀ and Aβ₁₋₄₂ between the finalization and beginning of each of the two treatment periods, changes in CSF levels of T-tau and P-tau throughout the study, assesment of structural changes in hippocampal volume, posterior cingulate volume, and othe areas if interest as shown by Magnetic Resonance Imaging (MRI), assessment of functional changes in the brain as detected by positron emission tomografy with 18F-fludeoxyglucose were also measured.

### Specific methodology for cognition/behavior assessment

The ADAS-Cog is an instrument specifically designed to evaluate the severity of the fundamental alterations in cognitive and behavioral function that are characteristic of patients with AD. Functional ability is assessed by means of the ADCS-ADL test, which offers detailed descriptions of each activity and requests the informer to describe the actions or behaviors observed, as explained above.

### Safety considerations

PE is a safe technique that may induce expected and therefore preventable and controllable, adverse reactions (e.g., hypocalcemia, hypotension). Considering the special vulnerability of the patients studied, to minimize the risks vital signs and laboratory test parameters were monitored more frequently than in typical clinical setting. In addition, patients were required to remain in the center before and after the PE procedure for longer periods of time than usual, and the caregiver was present with the exception of the actual procedure to maintain its blinded character. On rare occasions, agitated or anxious patients were accompanied by the caregiver during the procedure to ease patient's anxiety. However, the blinding protocol was maintained for both the patient and the caregiver.

The primary criterion of safety assessment was the percentage of TPE and LVPE procedures (including the infusion of albumin and IVIG) associated with at least one adverse event (AE) that may be related to the study procedure (adverse reaction). In addition, vital signs (blood pressure, heart rate, respiration rate, and body temperature) and laboratory test parameters (blood cell counts, platelet count, prothrombin time [Quick], activated partial thromboplastin time [aPTT], fibrinogen, total proteins, and calcium) were recorded before, during and after each PE session. The AEs were coded according to the adverse events classification of the World Health Organization (WHO) (MeDRA current version), and were described by a synonym (Lowest Level Term) and the affected organ/system, the intensity, causality and seriousness.

### Results

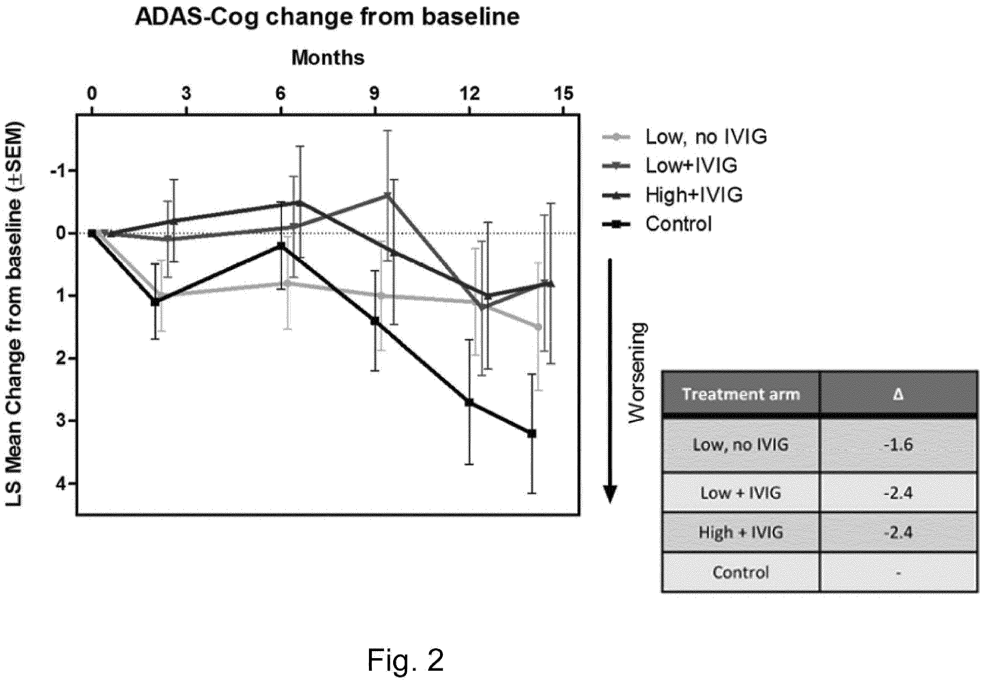

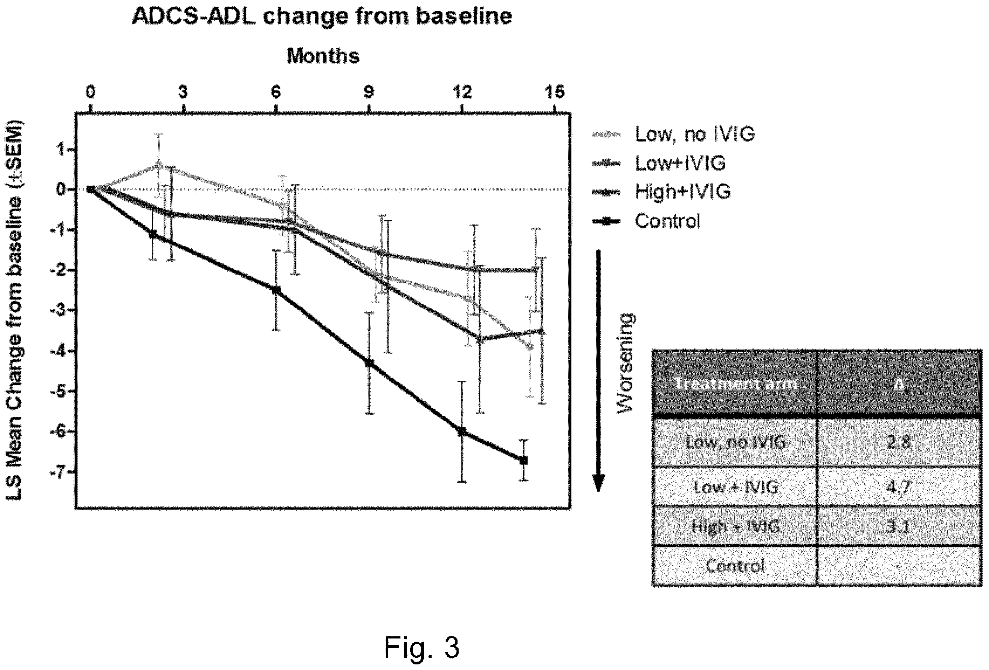

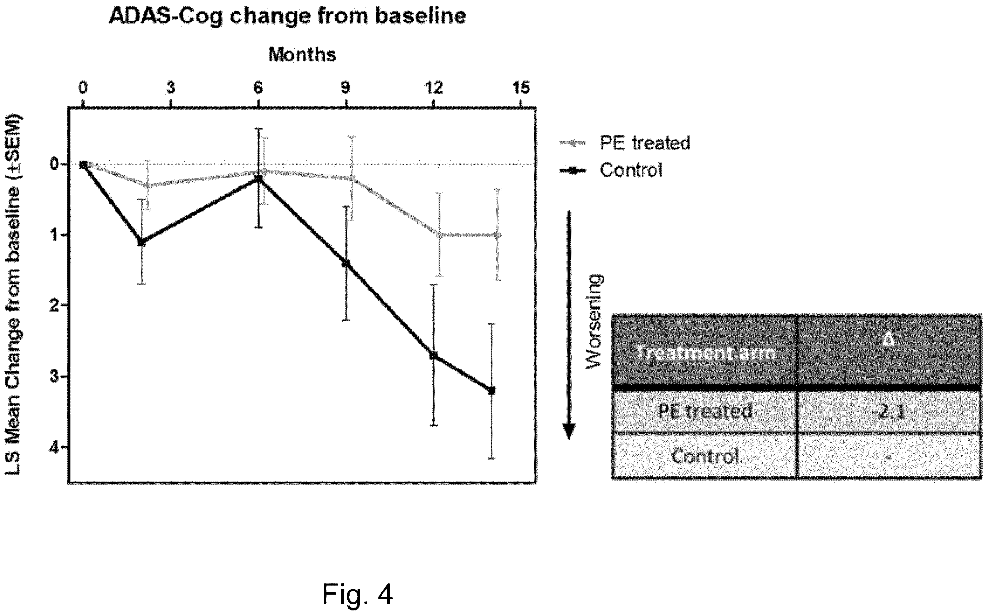

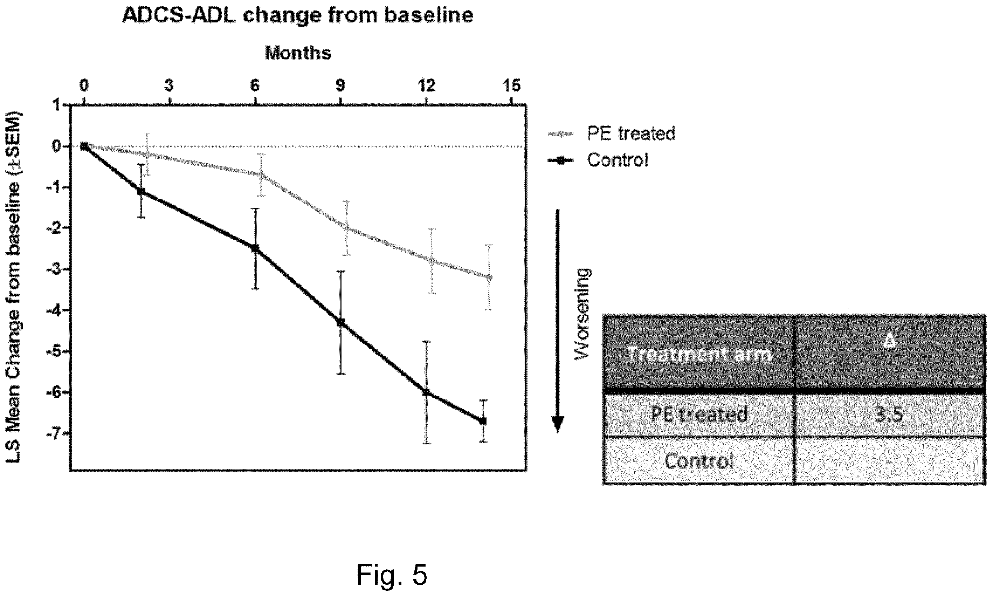

The changes from baseline of the ADAS-Cog scores and the ADCS-ADL inventory were analyzed over time using a mixed model for repeated measures (MMRM) approach. Efficacy was determined by the change in the total ADAS-Cog score and in the ADCS-ADL inventory score from baseline to 14 months follow up. As shown in Figure 2, the change of ADAS-Cog in the three dose groups (low albumin with no IGIV; low albumin with IGIV; and high albumin with IGIV) was lower that the control group (-1.6; -2.4; and -2.4, respectively) after 14 months of treatment. The same behaviour is observed for ADCS-ADL (see Figure 3), (-2.8; -4.7; and 3.1; respectively). No statistic difference was observed between all the treated groups. Figures 4 and 5 show a graph in which the treated groups were grouped and compared with the control group. A change from baseline of 2.1 and 3.5 of ADAS-Cog and ADCS-ADL, respectively, was observed.

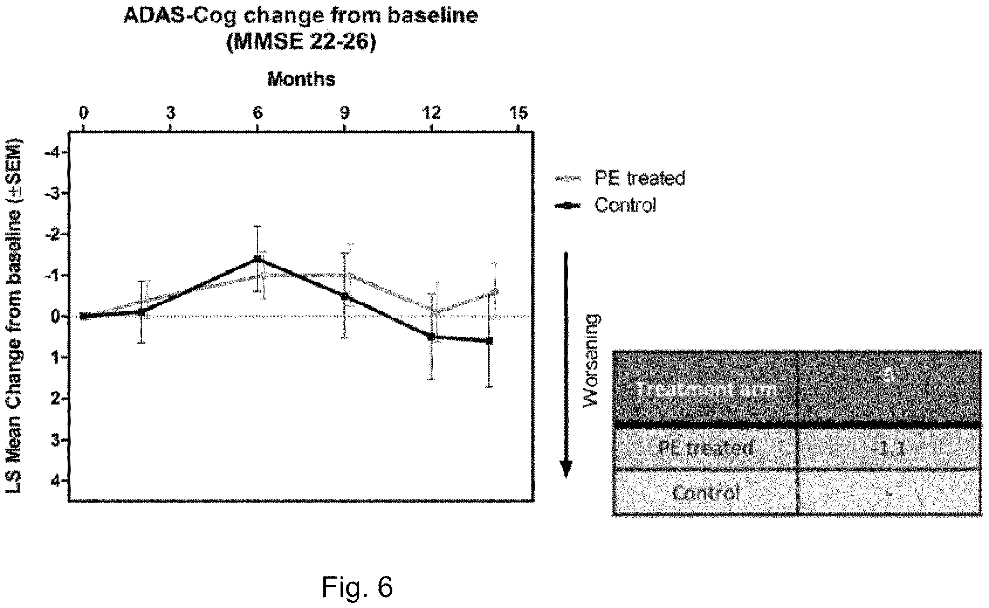

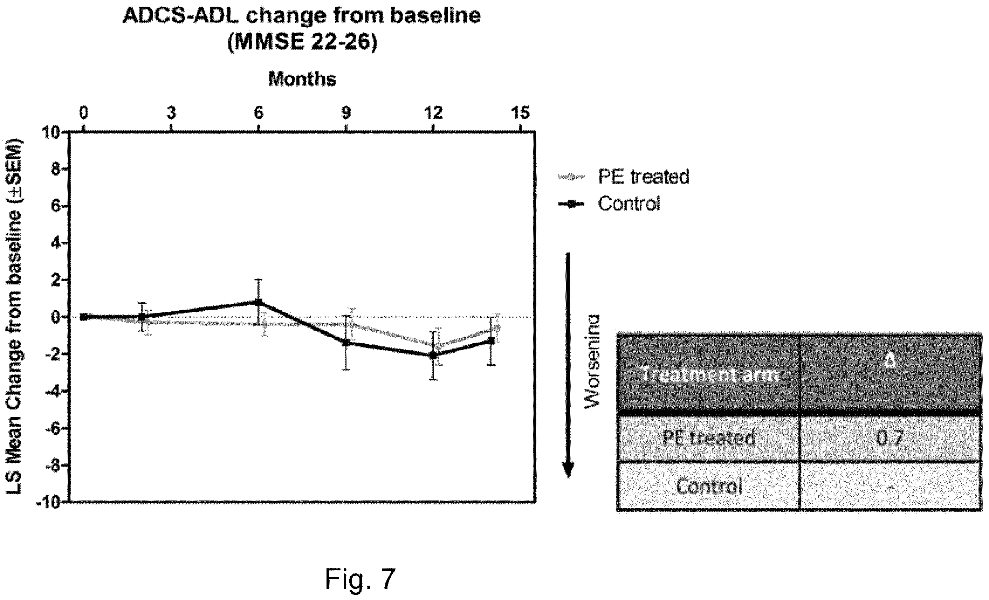

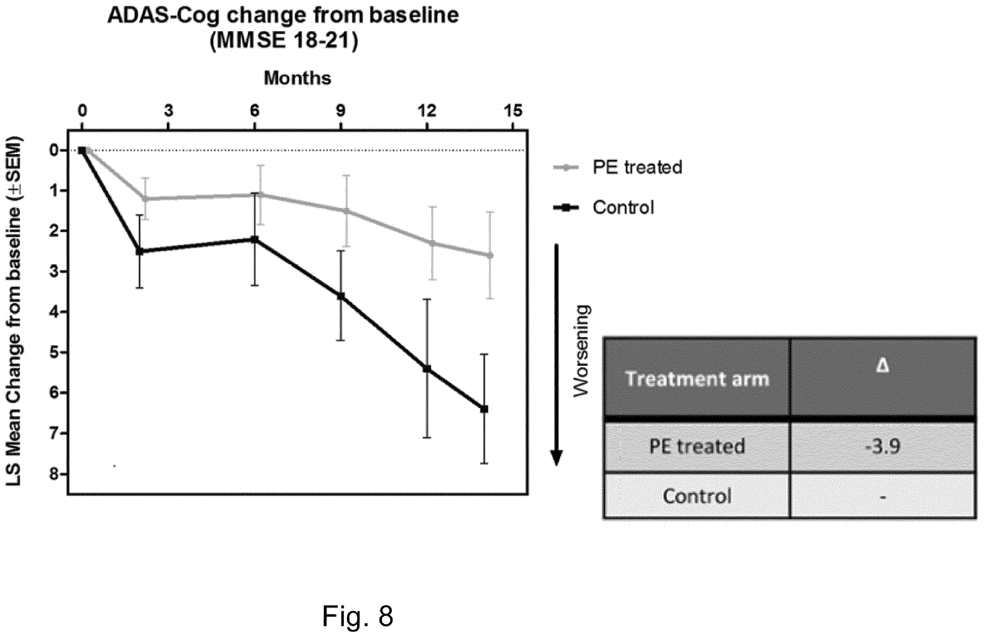

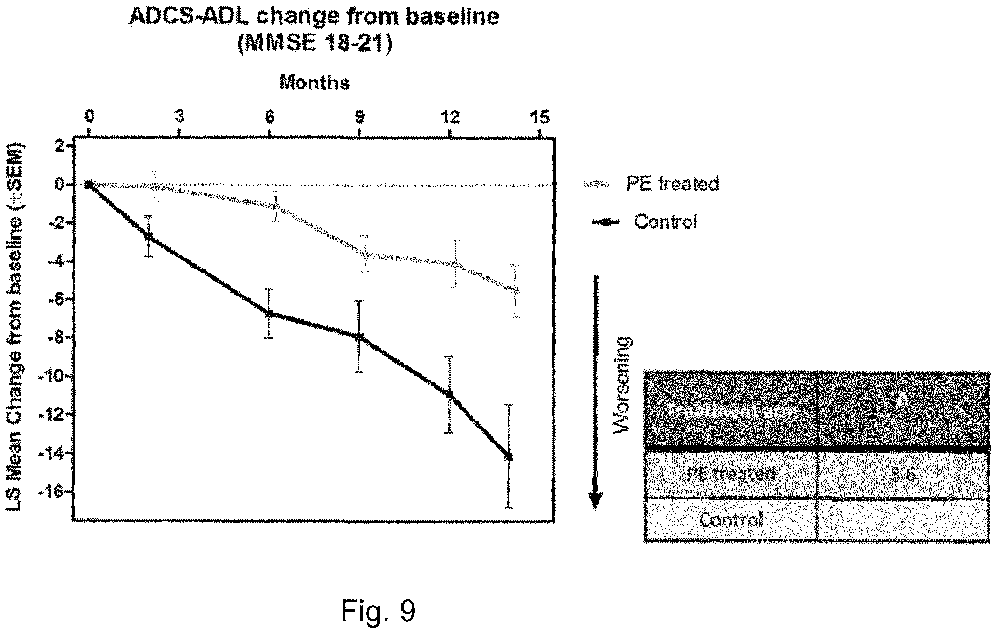

In addition to the above results, as shown in Figure 6 and 7, treated patients with mild AD (MMSE 22-26) were compared with the control group. A change from baseline of 1.1 and 0.7 of ADAS-Cog and ADCS-ADL, respectively, was observed. However, as shown in Figure 8 and 9, change from baseline of ADAS-Cog and ADCS-ADL in treated patients with moderate AD (MMSE 18-21) was of 3.9 and 8.6, respectively. This is a very surprising result, as it suggest that better results can be obtained in more advance stages of AD.

A modification was observed for plasma Aβ₁₋₄₂ levels which were lower in the PE-treated group after each treatment period (P < 0.05), and for CSF Aβ₁₋₄₂ levels (marginal P = 0.072 after the last PE compared with the baseline).

Moreover, neuroimaging studies confirmed that PE-treated patients has less hypoperfusion than controls in frontal, temporal, and parietal areas, and perfusion stabilization in Brodmann area BA38-R during the PE treatment period (P < 0.05); BA38 is assumed to play a significat role in language.

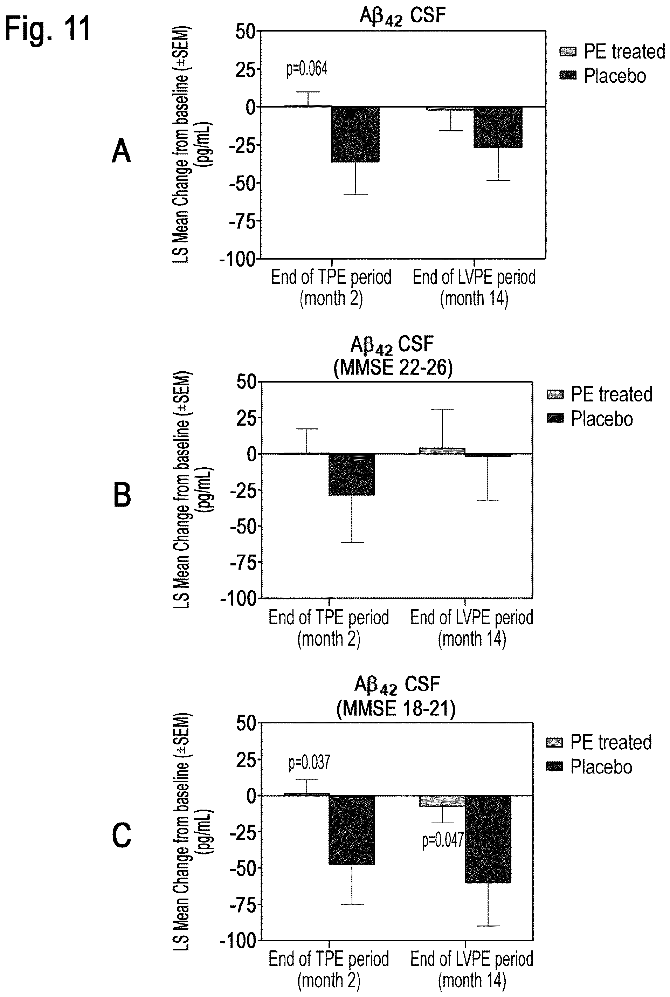

On the other hand, as shown in figure 11, changes of Aβ₁₋₄₂ levels in CSF at the end of each of the two treatment periods was demonstrated.

Figure 11A shows that Aβ₁₋₄₂ levels in CSF decrease in all treated with conventional therapeutic plasma exchange (TPE) and low volume plasma exchange (LVPE) patients as compared to non-treated patients (placebo group) (N=299). Figure 11B shows that Aβ₁₋₄₂ levels in CSF decrease in patients with mild AD (MMSE 22-26) treated with TPE as compared to non-treated patients (N=145). However, figure 11B shows that Aβ₁₋₄₂ levels in CSF is mantained in patients with mild AD (MMSE 22-26) treated with LVPE as compared to non-treated patients (N=145). Finally, figure 11C shows that Aβ₁₋₄₂ levels in CSF decrease in patients with moderate AD (MMSE 18-21) treated with TPE and LVPE as compared to non-treated patients (N=154).

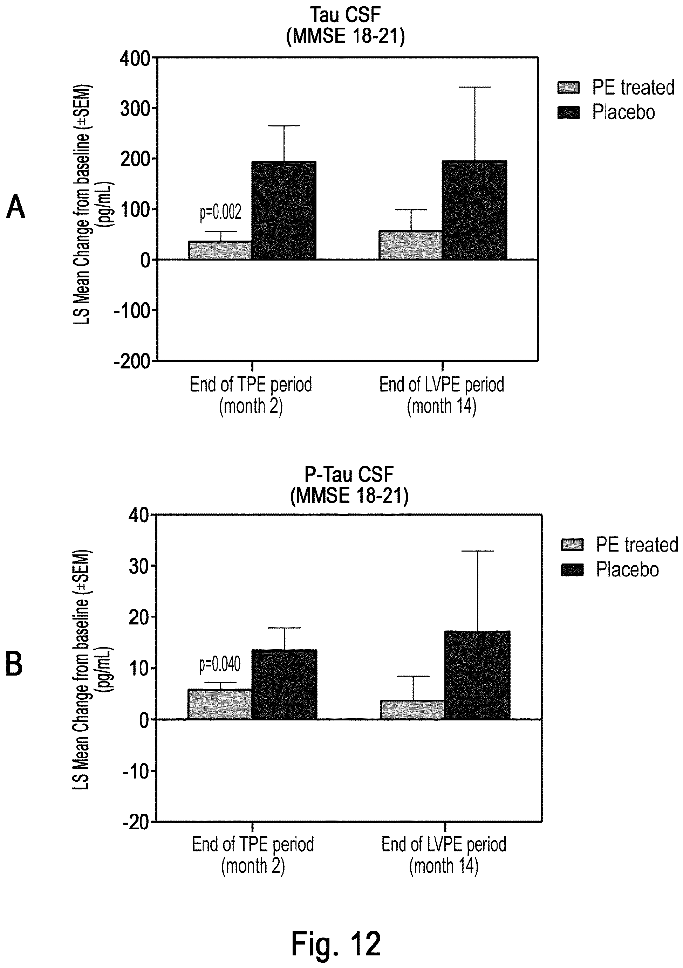

Moreover, as shown in figure 12, changes of T-tau and P-tau levels in CSF at the end of each of the two treatment periods was demonstrated.

Figure 12 shows that T-tau (Figure 12A) and P-tau (Figure 12B) levels in CSF decrease in patients with moderate AD (MMSE 18-21) treated with TPE and LVPE as compared to non-treated patients (placebo group) (N=154).

## Claims

1. Composition comprising human albumin at a concentration between 5% (w/v) and 25% (w/v) for use in the treatment of moderate Alzheimer's Disease (AD) by low-volume plasma exchange (LVPE), wherein before the treatment regime of LVPE previous rounds of conventional therapeutic plasma exchange (TPE) are carried out at a frequency of 1 TPE per week at least during 6 weeks and wherein each subsequent round of LVPE is conducted 30 days after the previous round, wherein the LVPE regime comprises 12 rounds, and wherein in the TPE regime plasma is replaced with the same volume of albumin at 5% (w/v) and during the LVPE regime plasma was replaced with albumin at 20% (w/v).

2. Composition for use, according to claim 1, **characterized in that** said LVPE regime involves blood volumes between 600 mL - 900 mL.

3. Composition for use, according to any of the preceding claims, **characterized in that** between 10 g and 40 g of albumin are used for replacement in each round of LVPE.

4. Composition for use, according to claim 3, **characterized in that** between between 20 g to 40 g of albumin are used for replacement in each round of LVPE.

5. Composition for use, according to any one of the preceding claims, **characterized in that** said treatment comprise in addition the administration of intravenous immunoglobulins (IGIV).

6. Composition for use, according to claim 5, **characterized in that** between 10 g and 20 g of immunoglobulins are used for replacement in some round of LVPE but never administered at the same time as albumin.

## Patentansprüche

1. Zusammensetzung umfassend Humanalbumin in einer Konzentration zwischen 5 % (w/v) und 25 % (w/v) zur Verwendung bei der Behandlung der mittelschweren Alzheimer-Krankheit (AD) durch Niedervolumen-Plasmaaustausch (LVPE), wobei vor dem LVPE-Behandlungsregime vorherige Runden eines konventionellen therapeutischen Plasmaaustauschs (TPE) mit einer Häufigkeit von mindestens 1 TPE pro Woche über einen Zeitraum von mindestens 6 Wochen durchgeführt werden und wobei jede nachfolgende LVPE-Runde 30 Tage nach der vorherigen Runde erfolgt, wobei das LVPE-Regime 12 Runden umfasst, und wobei im TPE-Regime Plasma durch das gleiche Volumen an Albumin zu 5 % (w/v) ersetzt wird und während des LVPE-Regimes das Plasma durch Albumin zu 20 % (w/v) ersetzt wurde.

2. Zusammensetzung zur Verwendung gemäß Anspruch 1, **dadurch gekennzeichnet, dass** das LVPE-Regime Blutvolumina zwischen 600 ml und 900 ml umfasst.

3. Zusammensetzung zur Verwendung gemäß einem der vorstehenden Ansprüche, **dadurch gekennzeichnet, dass** zwischen 10 g und 40 g Albumin als Ersatz in jeder LVPE-Runde verwendet werden.

4. Zusammensetzung zur Verwendung gemäß Anspruch 3, **dadurch gekennzeichnet, dass** zwischen 20 g und 40 g Albumin als Ersatz in jeder LVPE-Runde verwendet werden.

5. Zusammensetzung zur Verwendung gemäß einem der vorstehenden Ansprüche, **dadurch gekennzeichnet, dass** die Behandlung zusätzlich die Verabreichung von intravenösen Immunglobulinen (IGIV) umfasst.

6. Zusammensetzung zur Verwendung gemäß Anspruch 5, **dadurch gekennzeichnet, dass** zwischen 10 g und 20 g Immunglobuline zum Ersatz in einer LVPE-Runde verwendet werden, jedoch niemals gleichzeitig mit Albumin verabreicht werden.

## Revendications

1. Composition comprenant de l'albumine humaine à une concentration comprise entre 5 % (p/v) et 25 % (p/v) pour l'utilisation dans le traitement de la maladie d'Alzheimer modérée (AD) par échange plasmatique à faible volume (LVPE), dans laquelle, avant le régime de traitement par LVPE, des cycles précédents d'échange de plasma thérapeutique conventionnel (TPE) sont effectués à une fréquence de 1 TPE par semaine pendant au moins 6 semaines et dans laquelle chaque cycle ultérieur de LVPE est effectué 30 jours après le cycle précédent, dans laquelle le régime de LVPE comprend 12 cycles, et dans laquelle, dans le régime TPE, le plasma est remplacé par le même volume d'albumine à 5 % (p/v) et, pendant le régime de LVPE, le plasma est remplacé par de l'albumine à 20 % (p/v).

2. Composition pour utilisation selon la revendication 1, **caractérisée en ce que** le régime LVPE implique des volumes de sang compris entre 600 et 900 ml.

3. Composition pour utilisation selon l'une quelconque des revendications précédentes, **caractérisée en ce qu'**entre 10 g et 40 g d'albumine sont utilisés pour le remplacement dans chaque cycle de LVPE.

4. Composition pour utilisation selon la revendication 3, **caractérisée en ce qu'**entre 20 g et 40 g d'albumine sont utilisés pour le remplacement dans chaque cycle de LVPE.

5. Composition pour utilisation, selon l'une quelconque des revendications précédentes, **caractérisée en ce que** ledit traitement comprend en outre l'administration d'immunoglobulines intraveineuses (IGIV).

6. Composition pour utilisation, selon la revendication 5, **caractérisée en ce qu'**entre 10 g et 20 g d'immunoglobulines sont utilisés pour le remplacement dans certains cycles de LVPE mais jamais administrés en même temps que l'albumine.
